# EUROPEAN PATENT SPECIFICATION

(11) **EP 3 887 546 B1**
(45) Date of publication and mention of the grant of the patent: **21.02.2024**
(21) Application number: 20864670.3
(22) Date of filing: 29.04.2020
(51) Int. Cl.: C12Q 1/6883

(54) **BIOMARKERS FOR NEURODEGENERATIVE DISEASES**
BIOMARKER FÜR NEURODEGENERATIVE ERKRANKUNGEN
BIOMARQUEURS POUR MALADIES NEURODÉGÉNÉRATIVES

(30) Priority: 16.09.2019 AU 2019903434
(43) Date of publication of application: 06.10.2021
(73) Proprietor: Genieus Genomics Pty Ltd, Darlinghurst, New South Wales 2010 (AU)
(72) Inventor: KEON, Matt, Darlinghurst, New South Wales 2010 (AU); SAKSENA, Nitin, Darlinghurst, New South Wales 2010 (AU); BRENNAN, Sam, Darlinghurst, New South Wales 2010 (AU)
(74) Representative: Leonard, Thomas Charles
(86) International application number: PCT/AU2020/050423
(87) International publication number: WO 2021/051154

(56) References cited:
- WO-A1-2013/055865
- WO-A1-2017/186719
- WO-A2-2013/124816
- US-B2- 10 138 520
- JOILIN GREIG ET AL: "An Overview of MicroRNAs as Biomarkers of ALS", FRONTIERS IN NEUROLOGY, vol. 10, 7 March 2019 (2019-03-07), XP055805938, DOI: 10.3389/fneur.2019.00186
- RAHEJA RADHIKA ET AL: "Correlating serum micrornas and clinical parameters in amyotrophic lateral sclerosis", MUSCLE AND NERVE, vol. 58, no. 2, 1 August 2018 (2018-08-01) , pages 261-269, XP055805949, Hoboken, USA ISSN: 0148-639X, DOI: 10.1002/mus.26106 Retrieved from the Internet: URL:https://www.ncbi.nlm.nih.gov/pmc/artic les/PMC6103911/pdf/nihms948363.pdf>
- RICCI CLAUDIA ET AL: "MicroRNAs as Biomarkers in Amyotrophic Lateral Sclerosis", CELLS, vol. 7, no. 11, 20 November 2018 (2018-11-20), page 219, XP055943572, DOI: 10.3390/cells7110219
- BRENNAN SAMUEL ET AL: "Panoramic Visualization of Circulating MicroRNAs Across Neurodegenerative Diseases in Humans", MOLECULAR NEUROBIOLOGY, SPRINGER US, NEW YORK, vol. 56, no. 11, 29 April 2019 (2019-04-29), pages 7380-7407, XP036919796, ISSN: 0893-7648, DOI: 10.1007/S12035-019-1615-1 [retrieved on 2019-04-29]
- JOILIN Greig, Leigh P. Nigel, Newbury Sarah F., Hafezparast Majid: "An Overview of MicroRNAs as Biomarkers of ALS", Front Neurol. 2019, vol. 10, 7 March 2019 (2019-03-07), pages 1-9, XP055805938, DOI: 10.3389/fneur.2019.00186
- GROEN Kira, Maltby Vicki E., Lea Rodney A., Sanders Katherine A., Fink J. Lynn, Scott Rodney J., Tajouri Lotti, Lechner-Scott Jean: "Erythrocyte microRNA sequencing reveals differential expression in relapsing-remitting multiple sclerosis", BMC Med Genomics . 2018, vol. 11, no. 1, 21 May 2018 (2018-05-21), pages 1-12, XP055805940, DOI: 10.1186/s12920-018-0365-7
- SERAFIN A et al.: "Overexpression of blood microRNAs 103a, 30b, and 29a in L-dopa-treated patients with PD", Neurology, vol. 84, no. 7, 2015, pages 645-653, XP055546789, DOI: 10.1212/WNL.0000000000001258
- NAGARAJ Siranjeevi, Laskowska-Kaszub Katarzyna, D bski Konrad J., Wojsiat Joanna, D browski Micha , Gabryelewicz Tomasz, Ku nicki: "Profile of 6 microRNA in blood plasma distinguish early stage Alzheimer's disease patients from non-demented subjects", Oncotarget, vol. 8, no. 10, 2017, pages 16122-16143, XP055805943, DOI: 10.18632/oncotarget.15109
- SONG Yunping, Hu Mei, Zhang Jian, Teng Zhao-Qian, Chen Chu: "A novel mechanism of synaptic and cognitive impairments mediated via microRNA-30b in Alzheimer's disease", EBioMedicine, vol. 39, 2019, pages 409-421, XP055805945, DOI: 10.1016/j.ebiom.2018.11.059
- MARTINS Madalena, Alexandra Rosa; Leonor C Guedes; Benedita V Fonseca; Kristina Gotovac; Sara Violante; Tiago Mestre; Miguel Coelh: "Convergence of miRNA expression profiling, a- synuclein interaction and GWAS in Parkinson's disease", PLoS One, vol. 6, no. 10, 2011, pages 1-11, XP055080919, DOI: 10.1371/journal.pone.0025443
- RAHEJA Radhika, Regev Keren, Healy Brian C., Mazzola Maria Antonietta, Beynon Vanessa, Von Glehn Felipe, Paul Anu, Diaz cruz Camil: "Correlating serum micromas and clinical parameters in amyotrophic lateral sclerosis", Muscle Nerve, vol. 58, no. 2, August 2018 (2018-08), pages 261-269, XP055805949, DOI: 10.1002/mus.26106

## Description

### Cross-reference to Related Applications

The present application claims priority from Australian Provisional Patent Application No 2019903434 filed on 16 September 2019.

### Technical Field

The present disclosure relates to the field of biomarkers and their use in methods of diagnosing neurodegenerative diseases.

### Background

Neurodegenerative diseases (NDs) such as Alzheimer's disease (AD), Parkinson's disease (PD), Multiple sclerosis (MS), Amyotrophic Lateral Sclerosis (ALS) and dementia pose one of the greatest health challenges this century. NDs are a set of devastating, progressive diseases defined by neuron loss or irreversible dysfunction. Sporadic NDs are those with poorly defined causes or underlying pathophysiology, or those that show extreme variability in disease presentation and progression. An important unmet need is for sensitive and accurate diagnostic techniques capable of early detection of these NDs, and there is a considerable lack of genomic biomarkers to allow their detection in order to allow early intervention to facilitate improved clinical outcomes.

Joilin Greig et al ("An Overview of MicroRNAs as Biomarkers of ALS", Frontiers in Neurology, vol. 10, 7 March 2019) disclose microRNAs markers of ALS.

### Summary

The present disclosure is based on the surprising finding that the differential expression of a single biomarker, hsa-miR-30b-5p overlaps between Alzheimer's disease (AD), Parkinson's disease (PD), Multiple sclerosis (MS) and Amyotrophic Lateral Sclerosis (ALS). The inventors have demonstrated that the expression of hsa-miR-30b-5p is upregulated in the cerebrospinal fluid (CSF) of ALS patients. On the basis of these findings, the inventors have developed new methods of diagnosis of ALS as defined in the claims.

In another aspect, the present disclosure provides a method of classifying a subject as suffering from one of four neurodegenerative diseases, the method comprising measuring the level of expression of hsa-miR-30b-5p in the subject, wherein dysregulation of the level of expression of hsa-miR-30b-5p relative to the level of expression of hsa-miR-30b-5p in a healthy subject is indicative of the subject having one of four neurodegenerative diseases, wherein the four neurodegenerative diseases are Amyotrophic Lateral Sclerosis, Multiple Sclerosis, Alzheimer's disease or Parkinson's disease.

In another aspect, the present disclosure provides a method of reducing the number of potential neurodegenerative diseases a subject is suffering from in the diagnosis of a neurodegenerative disease, the method comprising measuring the level of expression of hsa-miR-30b-5p in the subject, wherein dysregulation of the level of expression of hsa-miR-30b-5p relative to the level of expression of hsa-miR-30b-5p in a healthy subject reduces the number of potential neurodegenerative diseases the subject is suffering from to a limited set of four, wherein said limited set of four diseases consists of Amyotrophic Lateral Sclerosis, Multiple Sclerosis, Alzheimer's disease and Parkinson's disease.

In another aspect, the present disclosure provides a method of selecting a subject for diagnosis of one of four neurodegenerative diseases, the method comprising measuring the level of expression of hsa-miR-30b-5p in the subject, wherein dysregulation of the level of expression of hsa-miR-30b-5p relative to the level of expression of hsa-miR-30b-5p in a healthy subject indicates that the subject should be selected for further diagnosis as potentially suffering from one of the four neurodegenerative diseases, wherein the four neurodegenerative diseases consist of Amyotrophic Lateral Sclerosis, Multiple Sclerosis, Alzheimer's disease and Parkinson's disease.

In another aspect, the present disclosure provides a method of diagnosing Amyotrophic Lateral Sclerosis, Multiple Sclerosis, Alzheimer's disease or Parkinson's disease in a subject, comprising measuring the level of expression of hsa-miR-30b-5p in the subject, wherein dysregulation of the expression of hsa-miR-30b-5p relative to the level of expression of hsa-miR-30b-5p in a healthy subject is indicative of the subject having Amyotrophic Lateral Sclerosis, Multiple Sclerosis, Alzheimer's disease or Parkinson's disease.

In another aspect, the present disclosure provides a method of diagnosing Amyotrophic Lateral Sclerosis in a subject, comprising measuring the level of expression of hsa-miR-30b-5p in the subject, wherein dysregulation of the expression of hsa-miR-30b-5p relative to the level of expression of hsa-miR-30b-5p in a healthy subject is indicative of the subject having Amyotrophic Lateral Sclerosis.

In another aspect, the present disclosure provides a method of diagnosing Multiple Sclerosis in a subject, comprising measuring the level of expression of hsa-miR-30b-5p in the subject, wherein dysregulation of the expression of hsa-miR-30b-5p relative to the level of expression of hsa-miR-30b-5p in a healthy subject is indicative of the subject having Multiple Sclerosis.

In another aspect, the present disclosure provides a method of diagnosing Alzheimer's disease in a subject, comprising measuring the level of expression of hsa-miR-30b-5p in the subject, wherein dysregulation of the expression of hsa-miR-30b-5p relative to the level of expression of hsa-miR-30b-5p in a healthy subject is indicative of the subject having Alzheimer's disease.

In another aspect, the present disclosure provides a method of diagnosing Parkinson's disease in a subject, comprising measuring the level of expression of hsa-miR-30b-5p in the subject, wherein dysregulation of the expression of hsa-miR-30b-5p relative to the level of expression of hsa-miR-30b-5p in a healthy subject is indicative of the subject having Parkinson's disease.

In another aspect, the present disclosure provides the use of a single biomarker to categorise a subject as suffering from any one or more of a limited set of four neurodegenerative diseases, wherein said limited set of four diseases consists of Amyotrophic Lateral Sclerosis, Multiple Sclerosis, Alzheimer's disease and Parkinson's disease, and wherein said single biomarker is hsa-miR-30b-5p. The use may further comprise measuring the level of expression of hsa-miR-30b-5p in the subject, wherein dysregulation of the level of expression of hsa-miR-30b-5p relative to the level of expression of hsa-miR-30b-5p in a healthy subject indicates that the subject is suffering from any one or more of Amyotrophic Lateral Sclerosis, Multiple Sclerosis, Alzheimer's disease and Parkinson's disease.

In the methods or the uses of the present disclosure, a decreased level of expression of hsa-miR-30b-5p in the subject relative to a healthy subject may be indicative that the subject has Amyotrophic Lateral Sclerosis, Alzheimer's disease or Parkinson's disease. This decreased level of expression may be determined in a bodily fluid sample taken from the subject. For example, the decreased level of expression may be determined in a blood and/or serum sample.

Any of the methods or the uses disclosed herein may comprise measuring the expression of hsa-miR-30b-5p in a subject's bodily fluid. The bodily fluid may be blood, serum, plasma, PBMCs or cerebrospinal fluid (CSF). In one example, the level of expression of hsa-miR-30b-5p is measured in the subject's blood. The level of expression of hsa-miR-30b-5p may be measured in any bodily fluid except for CSF. It has been determined by the inventors that the level of expression of hsa-miR-30b-5p may be decreased in blood and/or increased in CSF as an indicator of the subject suffering from ALS. Thus, in one example, the methods and uses disclosed herein may comprise measuring the level of expression of hsa-miR-30b-5p in the CSF of a subject (for example, in a sample of CSF derived from the subject).

In the methods or the uses of the present disclosure, an increased level of expression of hsa-miR-30b-5p in the subject relative to a healthy subject is indicative that the subject has Multiple Sclerosis.

The present invention provides a method of diagnosing Amyotrophic Lateral Sclerosis in a subject, the method comprising measuring the level of expression of hsa-miR-30b-5p in the cerebrospinal fluid (CSF) of the subject, wherein an increased level of expression of hsa-miR-30b-5p relative to the level of expression of hsa-miR-30b-5p in the CSF of a healthy subject is indicative of the subject having Amyotrophic Lateral Sclerosis. The level of expression of hsa-miR-30b-5p in the cerebrospinal fluid (CSF) is to be performed on a sample comprising CSF taken from the subject.

In another aspect, the present disclosure provides a method of diagnosing Amyotrophic Lateral Sclerosis in a subject, the method comprising measuring the level of expression of hsa-miR-30b-5p in the blood of the subject, wherein a decreased level of expression of hsa-miR-30b-5p relative to the level of expression of hsa-miR-30b-5p in the blood of a healthy subject is indicative of the subject having Amyotrophic Lateral Sclerosis. The level of expression of hsa-miR-30b-5p in the blood may be performed on a sample comprising blood taken from the subject.

In another aspect, the present disclosure provides a method of treating Amyotrophic Lateral Sclerosis, Multiple Sclerosis, Alzheimer's disease or Parkinson's disease comprising administering to a subject a regulator of hsa-miR-30b-5p expression. The regulator of hsa-miR-30b-5p expression may inhibit hsa-miR-30b-5p expression. For example, the regulator of hsa-miR-30b-5p expression reduces the level of hsa-miR-30b-5p in the cerebrospinal fluid (CSF) of the subject. The method may be a method of treating Amyotrophic Lateral Sclerosis. The regulator of hsa-miR-30b-5p expression may be an antisense oligonucleotide, a miRNA sponge, a miRNA mask or a small RNA inhibitor. Alternatively, the regulator of hsa-miR-30b-5p expression may increase hsa-miR-30b-5p expression.

Any of the diagnostic methods disclosed herein may be combined with the subsequent method of treatment of the diagnosed ND as disclosed herein. Methods of treatment are not part of the present invention.

In another aspect, the present disclosure provides the use of an inhibitor of hsa-miR-30b-5p expression in the manufacture of a medicament for treating Amyotrophic Lateral Sclerosis in a subject.

In another aspect, the present disclosure provides an inhibitor of hsa-miR-30b-5p expression for use in the treatment of Amyotrophic Lateral Sclerosis.

The inhibitor may be selected from the group consisting of an antisense oligonucleotide, a miRNA sponge, a miRNA mask or a small RNA inhibitor.

### Brief description of the drawings

The following figures form part of the present specification and are included to further demonstrate certain aspects of the present disclosure. The disclosure may be better understood by reference to one or more of these figures in combination with the detailed description of specific embodiments presented herein.
**Figure 1****. Proportion of differentially expressed miRNAs from various sources.** Pie chart showing the proportion of differentially expressed (DE) miRNAs derived from each source in various studies of ND. 49% of DE miRNAs were recorded in Serum, 27% in CSF, 11% in Plasma, 9% in PBMCs and 3% in whole blood.
**Figure 2****. Venn diagram of DE miRNAs in different compartments.**
   (a) 346 unique miRNAs were analysed from diverse compartments obtained from 72 different human studies on miRNAs in AD, PD, ALS (familial ALS (fALS) and sporadic ALS (sALS)) and MS. No DE miRNAs were discovered in all of these fluids and expression profiles were mostly unique to each fluid. The largest crossover exists between CSF and serum. (b) Venn diagram illustrating the relationships between 346 differentially expressed miRNAs between diverse NDs. hsa-miR-30b-5p was the single miRNA that was dysregulated in all four neurodegenerative diseases. The largest overlap was between fALS and sALS as these are clinical variants of a single pathology. The next largest overlap was between AD and PD.

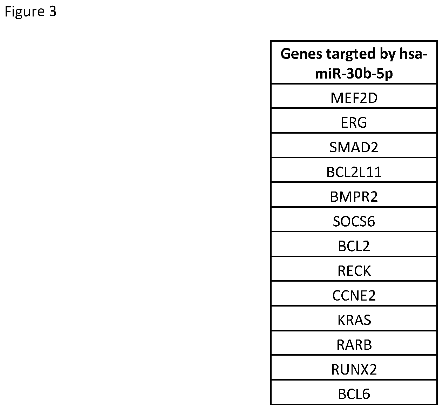

**Figure 3****. Gene targets of hsa-miR-30b-5p.**
   105 genes were identified to be predicted miRNA targets of at least two of 346 miRNAs which were differentially expressed in the same direction across the studies. hsa-miR-30b-5p targets 13 of these genes. Pathways targeted by hsa-miR-30b-5p include histone methylation, ERBB2 and wnt signalling, protein-folding and ubiquitination, mitochondrial function in apoptosis and necroptosis, and ion transport.

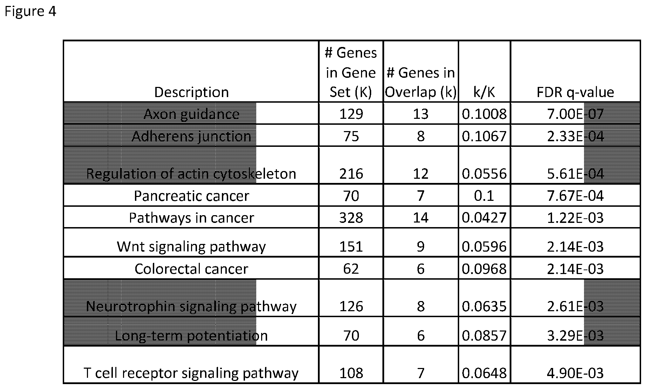

**Figure 4****. Functions of hsa-miR-30b-5p gene targets**
   Gene Set Enrichment Analysis (GSEA) analysis results of predicted hsa-miR-30b-5p targets. Pathways in grey are pathways that are implicated in neurodegenerative disease.

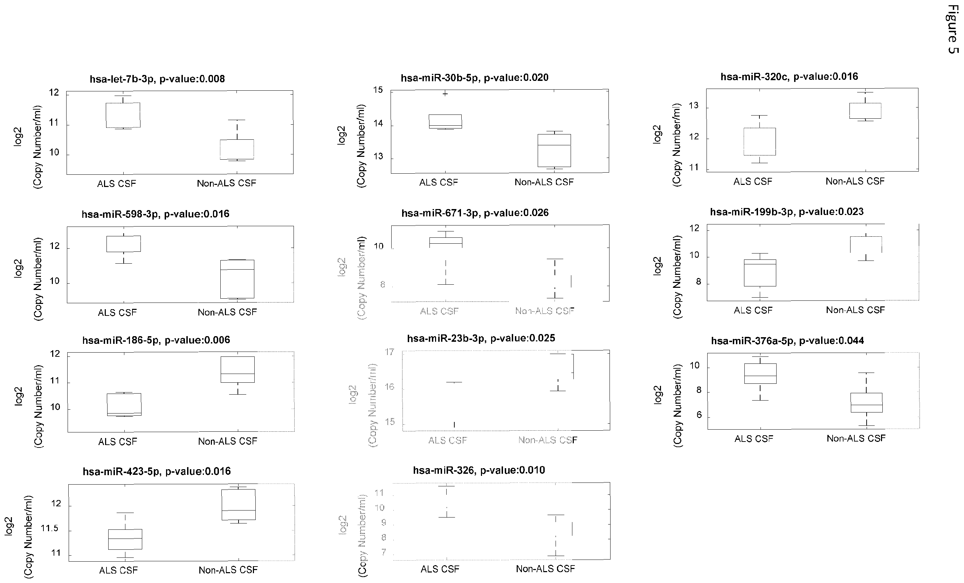

**Figure 5****. miRNAs with increased expression in ALS patients.**
   The expression of 11 miRNAs including hsa-miR-30b-5p are significantly increased in the CSF of ALS patients.

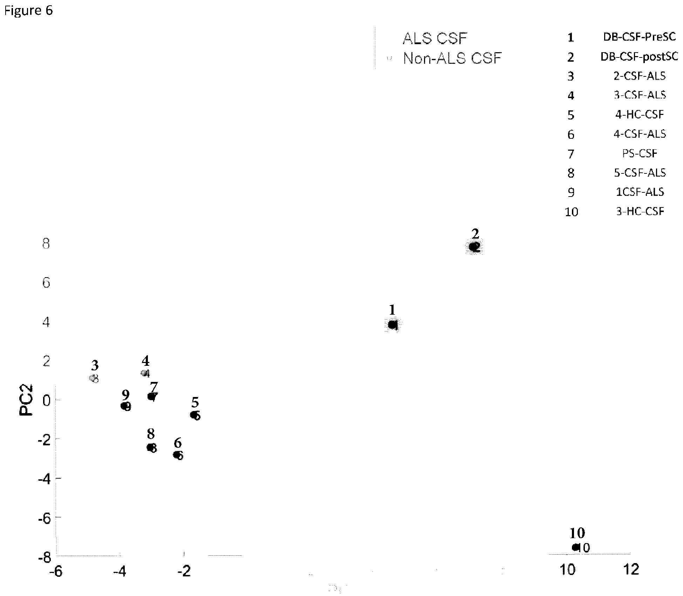

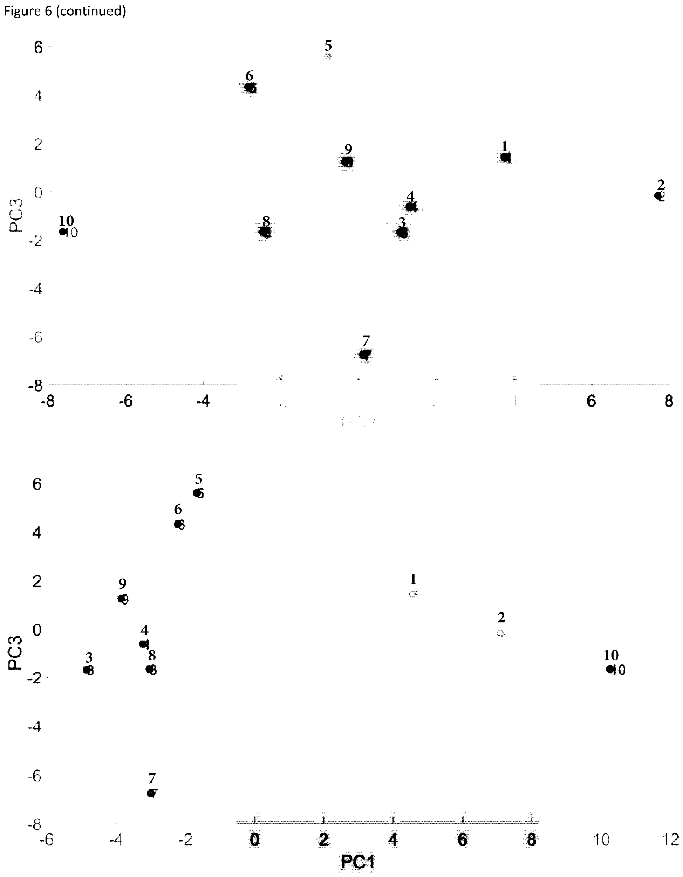

**Figure 6****. Principal component analysis (PCA) of the expression of hsa-miR-30b-5p in the CSF of ALS patients.**
   PCA of the expression of hsa-miR-30b-5p shows segregation between ALS patients (sample 3, 4, 6, 8 and 9) and healthy patients (sample 1, 2, 5, 7 and 10).

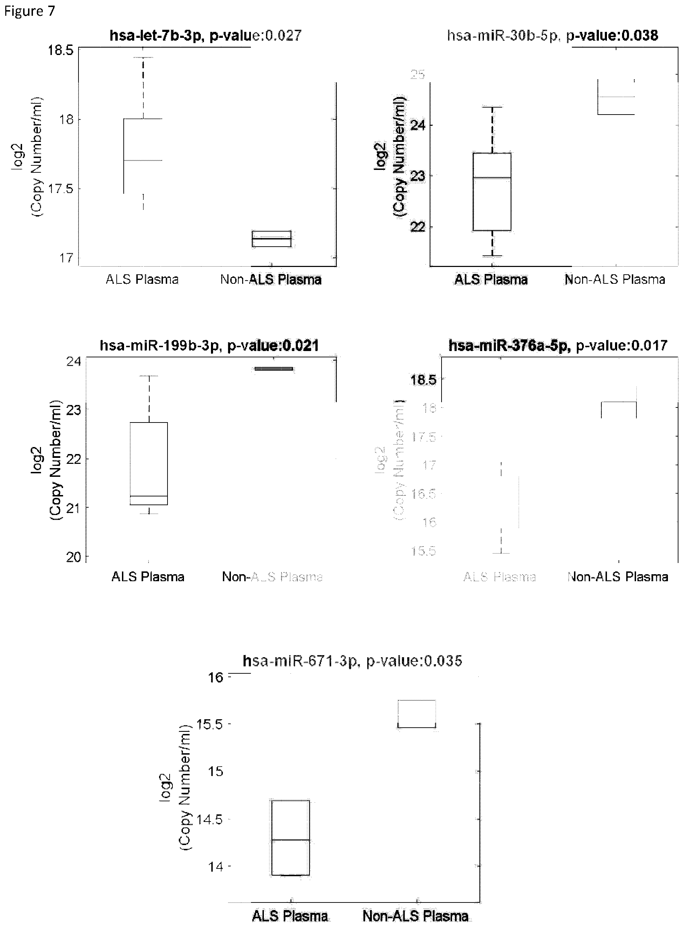

**Figure 7****. miRNAs with differential expression in the plasma ALS patients.**
   Differential expression of five miRNAs including hsa-miR-30b-5p in the plasma of ALS patients.

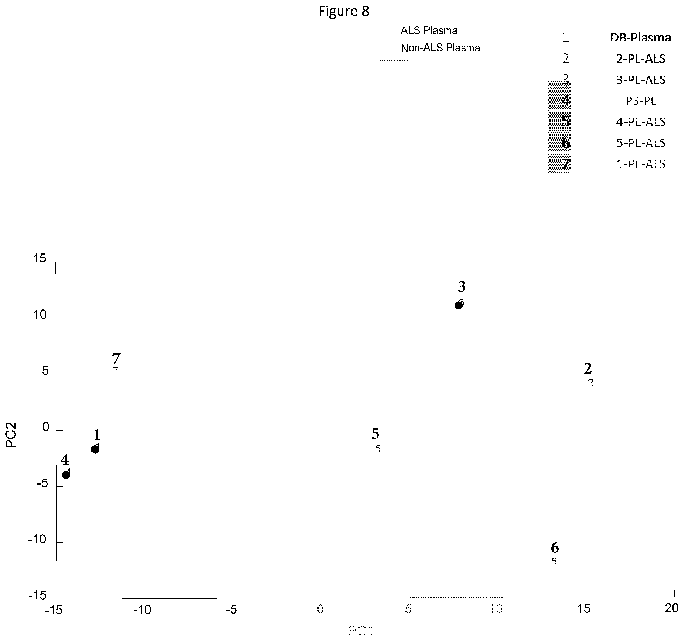

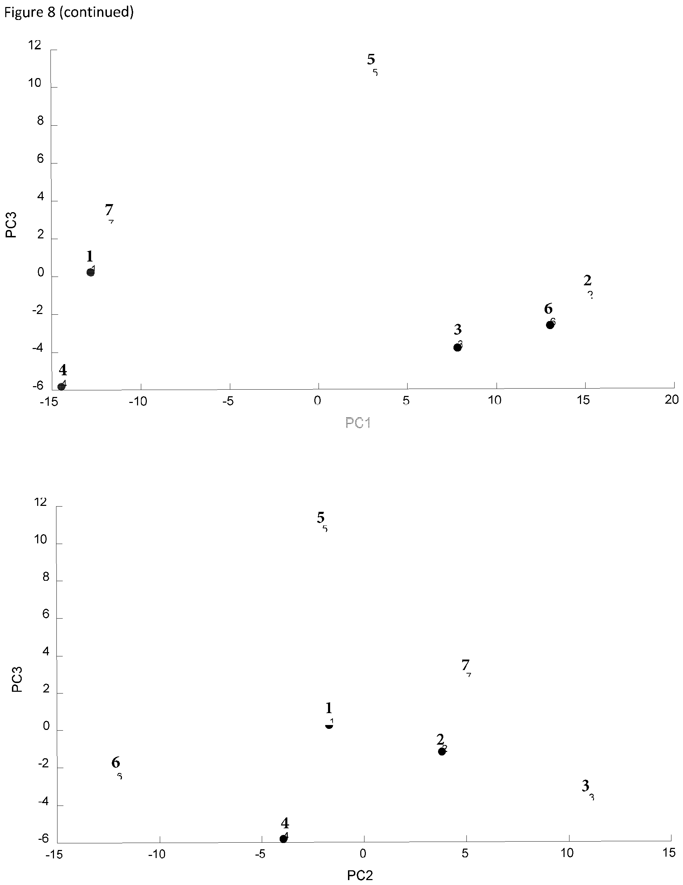

**Figure 8****. Principal component analysis (PCA) of the expression of hsa-miR-30b-5p in the plasma of ALS patients.**
   PCA of the expression of hsa-miR-30b-5p shows segregation between ALS patients (sample 2, 3, 5, 6 and 7) and healthy patients (sample 1 and 4).

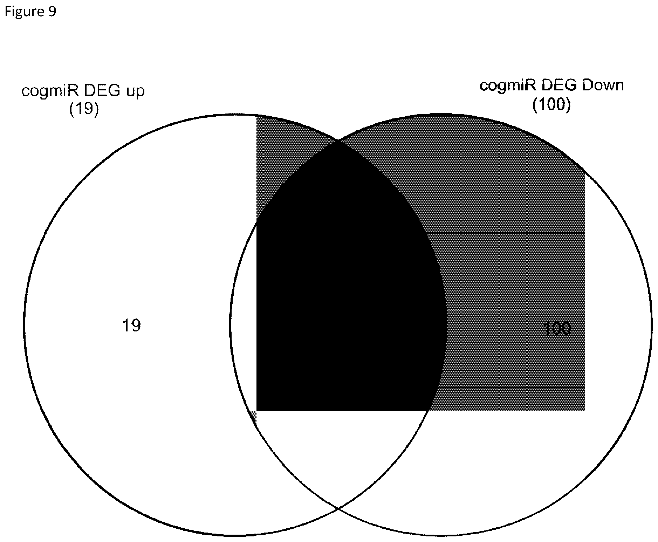

**Figure 9****. Venn diagram showing no overlapping miRNAs between those predicted to interact with down-regulated DEGs and up-regulated DEGs. The word "cogmiR" is a** contraction of "cognate miRNA".

### Key to the Sequence Listing

SEQ ID NO: 1 Nucleotide sequence for a reference human hsa-miR-30b-5p.
SEQ ID NO: 2 Exemplary miR-30b-5p inhibitor.

### Detailed description

### General Techniques and Definitions

Unless specifically defined otherwise, all technical and scientific terms used herein shall be taken to have the same meaning as commonly understood by one of ordinary skill in the art (e.g. in genomics, immunology, molecular biology, immunohistochemistry, biochemistry, oncology, and pharmacology).

The present disclosure is performed without undue experimentation using, unless otherwise indicated, conventional techniques of molecular biology, microbiology, recombinant DNA technology and immunology. Such procedures are described, for example in Sambrook, Fritsch & Maniatis, Molecular Cloning: A Laboratory Manual, Cold Spring Harbor Laboratories, New York, Fourth Edition (2012), whole of Vols I, II, and III; DNA Cloning: A Practical Approach, Vols. I and II (D. N. Glover, Second Edition., 1995), IRL Press, Oxford, whole of text; Oligonucleotide Synthesis: A Practical Approach (M. J. Gait, ed, 1984) IRL Press, Oxford, whole of text, and particularly the papers therein by Gait, ppl-22; Atkinson *et al,* pp35-81; Sproat *et al,* pp 83-115; and Wu *et al,* pp 135-151; 4. Nucleic Acid Hybridization: A Practical Approach (B. D. Hames & S. J. Higgins, eds., 1985) IRL Press, Oxford, whole of text; Immobilized Cells and Enzymes: A Practical Approach (1986) IRL Press, Oxford, whole of text; Perbal, B., A Practical Guide to Molecular Cloning (1984) and Methods In Enzymology (S. Colowick and N. Kaplan, eds., Academic Press, Inc.), whole of series.

Those skilled in the art will appreciate that the present disclosure is susceptible to variations and modifications other than those specifically described.

The present disclosure is not to be limited in scope by the specific embodiments described herein, which are intended for the purpose of exemplification only. Functionally equivalent products, compositions and methods are clearly within the scope of the disclosure, as described herein. The scope of the invention is defined by the appended claims.

Each feature of any particular aspect or embodiment or embodiment of the present disclosure may be applied *mutatis mutandis* to any other aspect or embodiment or embodiment of the present disclosure.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter. As used herein, the singular forms of "a", "and" and "the" include plural forms of these words, unless the context clearly dictates otherwise. For example, a reference to "a bacterium" includes a plurality of such bacteria, and a reference to "an allergen" is a reference to one or more allergens.

The term "and/or", e.g., "X and/or Y" shall be understood to mean either "X and Y" or "X or Y" and shall be taken to provide explicit support for both meanings or for either meaning.

Throughout this specification, the word "comprise' or variations such as "comprises" or "comprising" will be understood to imply the inclusion of a stated element, integer or step, or group of elements, integers or steps, but not the exclusion of any other element, integer or step, or group of elements, integers or steps.

### hsa-miR-30b-5p

hsa-miR-30b-5p has been shown to be highly abundant in the human neocortex alongside other members of the miRNA-30 family (Lukiw *et al.,* 2013). Moreover, hsa-miR-30b-5p has also been shown to correlate with neurofibrillary tangles in the CSF of patients with AD and PD, along with a significant fit across increasing plaque density stages (Burgos *et al.,* 2014). The sequence of hsa-miR-30b-5p is publicly available. An exemplary sequence is 5'-UGUAAACAUCCUACACUCAGCU-3' (SEQ ID NO: 1). The chromosomal location of hsa-miR-30b-5p has been described as NC_000008.11 (134800520..134800607, complement). An exemplary sequence of the seed sequence of hsa-miR-30b-5p is 5'-GUAAACA-3'.

Surprisingly, the inventors have found that hsa-miR-30b-5p is dysregulated in all four of the neurodegenerative diseases, Amyotrophic Lateral Sclerosis, Multiple Sclerosis, Alzheimer's disease and Parkinson's disease. Further, hsa-miR-30b-5p is involved in some of the most relevant pathways that underlie neurodegeneration and may thus serve a useful function as a biomarker. Accordingly, disclosed herein are new methods of subject classification, methods of diagnosis of neurodegenerative diseases and methods of treating neurodegenerative diseases.

It will be appreciated by the person skilled in the art that the level of expression of hsa-miR-30b-5p may be measured by any means. Suitable means for determining the level of expression of hsa-miR-30b-5p are known in the art. For example, see Zhang, L., Jia, X. "Down-regulation of miR-30b-5p protects cardiomyocytes against hypoxia-induced injury by targeting Aven." Cell Mol Biol Lett 24, 61 (2019).

The dysregulation of the level of expression of hsa-miR-30b-5p relative to the level of expression of hsa-miR-30b-5p in a healthy subject may be indicative that the subject is suffering from any one or more of Amyotrophic Lateral Sclerosis, Multiple Sclerosis, Alzheimer's disease and Parkinson's disease.

Measuring the level of hsa-miR-30b-5p expression in a subject's is within the means of a person skilled in the art. In accordance with the methods of the present disclosure, the level of hsa-miR-30b-5p expression may be measured by qPCR or by RNA seq or by using primers or probes that bind to hsa-miR-30b-5p. Suitable primers can be prepared by a person skilled in the art. See, for example, the hsa-miR-30b-5p forward and reverse primers disclosed in Zhang, L., Jia, X. Cell Mol Biol Lett 24, 61 (2019). The level of expression of hsa-miR-30b-5p in the subject may be compared to the level of expression of hsa-miR-30b-5p in a healthy subject by any method known in the art.

The expression of hsa-miR-30b-5p may be measured in a biological sample taken from the subject, for example in the subject's bodily fluids. Thus, the bodily fluid may be blood, serum, plasma, PBMCs or cerebrospinal fluid (CSF). In one example, the bodily fluid is blood. In another example, the bodily fluid is CSF. Any of the methods disclosed herein may comprise a step of taking a biological sample from a subject and determining the level expression of hsa-miR-30b-5p in the sample.

It will be understood by the person skilled in the art that "dysregulation of the level of the expression of " may be determined by comparing the level of expression of hsa-miR-30b-5p in the subject and comparing it to the level of expression of hsa-miR-30b-5p in a healthy subject or to a reference level that is indicative of the level of expression of hsa-miR-30b-5p in a healthy subject. Thus, any of the methods disclosed herein may comprise a step of establishing a reference level of hsa-miR-30b-5p expression. Alternatively, any of the methods disclosed herein may comprise a step of comparing a measurement of hsa-miR-30b-5p expression to a predetermined reference level. Suitable threshold levels can then be determined according to the particular methodology used to measure hsa-miR-30b-5p expression, which threshold levels can establish a significant difference in expression of hsa-miR-30b-5p and a healthy control, such a difference then being indicative of dysregulation of hsa-miR-30b-5p in the subject. It will be appreciated that the precise threshold levels will vary depending on the samples used to establish those threshold levels and according to the particular analytical methodology used in each instance. A "decreased" level of hsa-miR-30b-5p expression is a level of hsa-miR-30b-5p expression that is lower relative to the reference level of hsa-miR-30b-5p expression. Conversely, an "increased" level of hsa-miR-30b-5p expression is a level of hsa-miR-30b-5p expression that is greater than the reference level of hsa-miR-30b-5p expression. The "normal" level of hsa-miR-30b-5p expression or the reference level of hsa-miR-30b-5p expression may be determined by selecting any suitable biological sample from which to derive the level of hsa-miR-30b-5p expression in a non-disease state. The biological sample may be or may comprise a bodily fluid. The bodily fluid may blood, serum, plasma, PBMCs or cerebrospinal fluid (CSF). For example, the level of hsa-miR-30b-5p expression may be measured in CSF. The biological sample may be subject to any suitable pre-treatment steps before the measurement is performed, in order to improve the accuracy and/or efficiency of the measurement.

Also disclosed herein is a kit for determining level of hsa-miR-30b-5p expression in a subject. In one example, the kit comprises one or more reagents specifically configured to determine a level of expression of hsa-miR-30b-5p in a subject. The one or more reagents configured to determine a level of expression of hsa-miR-30b-5p in a subject may be primers or probes for qPCR to determine the level of hsa-miR-30b-5p expression. Thus, the kit may comprise one or more amplification primers that bind specifically to hsa-miR-30b-5p.

### Methods of diagnosing and/or treating neurodegenerative diseases and subject selection

The inventors have surprisingly shown for the first time that dysregulation in the level of expression of a single biomarker, hsa-miR-30b-5p is indicative that a subject is suffering from one of four neurodegenerative diseases. The four neurodegenerative diseases are Amyotrophic Lateral Sclerosis, Multiple Sclerosis, Alzheimer's disease and Parkinson's disease. Accordingly, the present disclosure provides methods of selecting and/or diagnosing and/or treating Amyotrophic Lateral sclerosis (ALS), Multiple Sclerosis (MS), Alzheimer's disease (AD) or Parkinson's disease (PD) in a subject. Furthermore, the inventors have shown that there is increased hsa-miR-30b-5p expression in the CSF of subjects suffering from ALS. In any of the methods disclosed herein, the level of expression of hsa-miR-30b-5p may indicate the extent of disease progression in the subject. As a result, the methods disclosed herein may further comprise a step of determining the likelihood of disease progression based on the level of expression of hsa-miR-30b-5p.

As used herein, the term "subject" refers to any animal, for example, a mammalian animal, including, but not limited to humans, non-human primates, livestock (e.g. sheep, horses, cattle, pigs, donkeys), companion animals (e.g. pets such as dogs and cats), laboratory test animals (e.g. mice, rabbits, rats, guinea pigs), performance animals (e.g. racehorses, camels, greyhounds) or captive wild animals. In one embodiment, the "subject" is a human. Typically, the terms "subject" and "patient" are used interchangeably, particularly in reference to a human subject. It will be understood by the person skilled in the art that as used herein, "the subject" is the test subject and a "healthy subject" is a control subject who is not suffering from Amyotrophic Lateral sclerosis (ALS), Multiple Sclerosis (MS), Alzheimer's disease (AD) or Parkinson's disease (PD).

In one example, the present disclosure provides a method of classifying a subject as suffering from one of four neurodegenerative diseases, the method comprising measuring the level of expression of hsa-miR-30b-5p in the subject, wherein dysregulation of the level of expression of hsa-miR-30b-5p relative to the level of expression of hsa-miR-30b-5p in a healthy subject is indicative of the subject having one of four neurodegenerative diseases, wherein the four neurodegenerative diseases are Amyotrophic Lateral Sclerosis, Multiple Sclerosis, Alzheimer's disease or Parkinson's disease.

In another example, the present disclosure provides a method of reducing the number of potential neurodegenerative diseases a subject is suffering from in the diagnosis of a neurodegenerative disease, the method comprising measuring the level of expression of hsa-miR-30b-5p in the subject, wherein dysregulation of the level of expression of hsa-miR-30b-5p relative to the level of expression of hsa-miR-30b-5p in a healthy subject reduces the number of potential neurodegenerative diseases the subject is suffering from to a limited set of four, wherein said limited set of four diseases consists of Amyotrophic Lateral Sclerosis, Multiple Sclerosis, Alzheimer's disease and Parkinson's disease.

In another example, the present disclosure provides a method of selecting a subject for diagnosis of one of four neurodegenerative diseases, the method comprising measuring the level of expression of hsa-miR-30b-5p in the subject, wherein dysregulation of the level of expression of hsa-miR-30b-5p relative to the level of expression of hsa-miR-30b-5p in a healthy subject indicates that the subject should be selected for further diagnosis as potentially suffering from one of the four neurodegenerative diseases, wherein the four neurodegenerative diseases consist of Amyotrophic Lateral Sclerosis, Multiple Sclerosis, Alzheimer's disease and Parkinson's disease.

In another example, the present disclosure provides a method of diagnosing Amyotrophic Lateral Sclerosis, Multiple Sclerosis, Alzheimer's disease or Parkinson's disease in a subject, comprising measuring the level of expression of hsa-miR-30b-5p in the subject, wherein dysregulation of the expression of hsa-miR-30b-5p relative to the level of expression of hsa-miR-30b-5p in a healthy subject is indicative of the subject having Amyotrophic Lateral Sclerosis, Multiple Sclerosis, Alzheimer's disease or Parkinson's disease. For example, the method may be a method of diagnosing Amyotrophic Lateral Sclerosis in a subject, the method comprising measuring the level of expression of hsa-miR-30b-5p in the blood of the subject, wherein a decreased level of expression of hsa-miR-30b-5p relative to the level of expression of hsa-miR-30b-5p in the blood of a healthy subject is indicative of the subject having Amyotrophic Lateral Sclerosis.

In one embodiment, there is provided a method of diagnosing Amyotrophic Lateral sclerosis, Multiple Sclerosis, Alzheimer's disease or Parkinson's disease in a subject, comprising measuring the level of expression of hsa-miR-30b-5p in the subject and comparing it to the level of expression of hsa-miR-30b-5p in a healthy subject, wherein the dysregulation of the expression of hsa-miR-30b-5p is indicative of the subject having Amyotrophic Lateral sclerosis, Multiple Sclerosis, Alzheimer's disease or Parkinson's disease.

In one embodiment, a decreased level of expression of hsa-miR-30b-5p in the subject relative to a healthy subject is indicative that the subject has Amyotrophic Lateral sclerosis, Alzheimer's disease or Parkinson's disease.

In another embodiment, an increased level of expression of hsa-miR-30b-5p in the subject relative to a healthy subject is indicative that the subject has Multiple Sclerosis.

The level of expression of hsa-miR-30b-5p may be measured in a subject's bodily fluid. Examples of suitable body fluids include, but are not limited to blood, serum, plasma, PBMCs or CSF. In one embodiment, the bodily fluid is CSF.

In another example, the present disclosure provides the use of a single biomarker to categorise a subject as suffering from any one or more of a limited set of four neurodegenerative diseases, wherein said limited set of four diseases consists of Amyotrophic Lateral Sclerosis, Multiple Sclerosis, Alzheimer's disease and Parkinson's disease, and wherein said single biomarker is hsa-miR-30b-5p. The level of expression of hsa-miR-30b-5p may be measured. The dysregulation of the level of expression of hsa-miR-30b-5p relative to the level of expression of hsa-miR-30b-5p in a healthy subject may be indicative that the subject is suffering from any one or more of Amyotrophic Lateral Sclerosis, Multiple Sclerosis, Alzheimer's disease and Parkinson's disease.

It will be understood by the person skilled in the art that a decreased level of expression of hsa-miR-30b-5p in the subject relative to a healthy subject is indicative that the subject has Amyotrophic Lateral Sclerosis, Alzheimer's disease or Parkinson's disease. It will also be understood by the person skilled in the art that an increased level of expression of hsa-miR-30b-5p in the subject relative to a healthy subject is indicative that the subject has Multiple Sclerosis.

As used herein, the terms "treating", "treat" or "treatment" and variations thereof, refer to clinical intervention designed to alter the natural course of the individual or cell being treated during the course of clinical pathology. Desirable effects of treatment include decreasing the rate of disease progression, ameliorating or palliating the disease state, and remission or improved prognosis. As used herein, the term "improved" shall be understood to mean decreased mortality, increased magnitude of response, decreased timing of treatment, decreased disease progression, decrease of pathological symptoms for the patient.

In one example, the present disclosure provides a method of treating Amyotrophic Lateral Sclerosis, Multiple Sclerosis, Alzheimer's disease or Parkinson's disease comprising administering to a subject a regulator of hsa-miR-30b-5p expression. The regulator may increase hsa-miR-30b-5p expression. Alternatively, the regulator may inhibit hsa-miR-30b-5p expression. Thus, the regulator may be an inhibitor of hsa-miR-30b-5p expression. Suitable examples of regulators include, but are not limited to, an antisense oligonucleotide, a miRNA sponge, a miRNA mask or a small RNA inhibitor. The regulator may reduce the level of expression of hsa-miR-30b-5p in the subject's bodily fluid, for example, in the subject's blood, serum, plasma, PBMCs or cerebrospinal fluid (CSF).

It will be understood by the person skilled in the art that when miRNA30b-5p expression is increased, an inhibitor of hsa-miR-30b-5p expression may be administered to the subject to decrease hsa-miR-30b-5p expression. Conversely, when hsa-miR-30b-5p expression is decreased, a regulator of hsa-miR-30b-5p expression may be administered to the subject to increase hsa-miR-30b-5p expression.

The methods disclosed herein may be a method of treating Amyotrophic Lateral Sclerosis. Thus, in one example, the method may be a method of treating Amyotrophic Lateral Sclerosis, comprising administering to a subject a regulator of hsa-miR-30b-5p expression, wherein the regulator of hsa-miR-30b-5p expression reduces the level of hsa-miR-30b-5p in the cerebrospinal fluid of the subject.

Disclosed herein is the use of an inhibitor of hsa-miR-30b-5p expression in the manufacture of a medicament for treating Amyotrophic Lateral Sclerosis in a subject. Also disclosed herein is an inhibitor for use in the treatment of Amyotrophic Lateral Sclerosis. Any suitable inhibitor may be used to inhibit hsa-miR-30b-5p expression. The inhibitor may target a seed sequence of hsa-miR-30b-5p. Inhibition of hsa-miR-30b-5p expression by the inhibitor may be partial or complete. The inhibition may be apparent relative to subject in which the expression of hsa-miR-30b-5p expression has not been inhibited. Methods of designing suitable inhibitors are known in the art. Suitable examples of inhibitors include, but are not limited to, DNA (gDNA, cDNA), RNA (sense RNAs, antisense RNAs, mRNAs, tRNAs, rRNAs, small interfering RNAs (siRNAs), short hairpin RNAs (shRNAs), micro RNAs (miRNAs), small nucleolar RNAs (snoRNAs), small nuclear RNAs (snRNAs), ribozymes, aptamers, DNAzymes, antisense oliogonucleotides, vectors, plasmids, other ribonuclease-type complexes, miRNA sponges, miRNA masks, small RNA inhibitors and mixtures thereof. The sequence of hsa-miR-30b-5p is publicly available and can be used to design suitable inhibitors by methods known in the art. A reference nucleotide sequence of hsa-miR-30b-5p is provided in SEQ ID NO: 1. For example, see Zhang, L., Jia, X. "Down-regulation of miR-30b-5p protects cardiomyocytes against hypoxia-induced injury by targeting Aven." Cell Mol Biol Lett 24, 61 (2019), which describes use of the miR-30b-5p inhibitor AGAACAGUGAAAUUUCCAGUCC (SEQ ID NO: 2). Means of introduction of the inhibitors to the subject are known in the art.

The medicament may be may also include excipients or agents such as solvents, diluents, dispersion media, coatings, antibacterial and antifungal agents, isotonic and absorption delaying agents and the like that are physiologically compatible and are not deleterious to the inhibitor as described herein or use thereof. The use of such carriers and agents to prepare compositions of pharmaceutically active substances is well known in the art (see, for example Remington: The Science and Practice of Pharmacy, 21st Edition; Lippincott Williams & Wilkins: Philadelphia, PA, 2005).

The medicament include those for oral, rectal, nasal, topical (including buccal and sublingual), parenteral administration (including intramuscular, intraperitoneal, sub-cutaneous and intravenous), or in a form suitable for administration by inhalation or insufflation.

The inhibitor of hsa-miR-30b-5p expression, together with a conventional adjuvant, carrier or diluent, may be placed into the form of pharmaceutical compositions and unit dosages thereof, and in such form may be employed as solids, such as tablets or filled capsules, or liquids as solutions, suspensions, emulsions, elixirs or capsules filled with the same, all for oral use, or in the form of sterile injectable solutions for parenteral (including subcutaneous) use.

The medicament for the administration of the inhibitors of this disclosure may conveniently be presented in dosage unit form and may be prepared by any of the methods well known in the art of pharmacy.

The medicament and methods disclosed herein may further comprise other therapeutically active compounds which are usually applied in the treatment of the disclosed disorders or conditions. Selection of the appropriate agents for use in
combination therapy may be made by one of ordinary skill in the art, according to conventional pharmaceutical principles. The combination of therapeutic agents may act synergistically to effect the treatment or prevention of the various disorders or conditions disclosed herein. Using this approach, one may be able to achieve therapeutic efficacy with lower dosages of each agent, thus reducing the potential for adverse side effects.

When other therapeutic agents are employed in combination with those disclosed herein, they may be used for example in amounts as noted in the Physician's Desk Reference (PDR) or as otherwise determined by one of ordinary skill in the art.

It will be understood, however, that the specific dose level and frequency of dosage for any particular subject may be varied and will depend upon a variety of factors including the activity of the specific compound employed, the metabolic stability and length of action of that compound, the age, body weight, general health, gender, diet, mode and time of administration, rate of excretion, drug combination, the severity of the particular condition, and the subject undergoing therapy.

The inhibitors or medicaments disclosed herein may be delivered to a subject by any suitable means.

### Examples

### Example 1. Differential expression of miRNAs in neurodegenerative disease

To determine the expression in body fluid of miRNAs which have been implicated in Alzheimer's Disease (AD), Parkinson's Disease (PD), Amyotrophic Lateral Sclerosis (ALS) and Multiple Sclerosis, a knowledge base was constructed by performing multiple literature searches using various permutations and combinations of search terms encompassing AD, PD, ALS or MS on the NCBI, Pubmed and Google Scholar platforms. The search terms used were "miRNA biomarker (AD, PD, ALS or MS)" on the NCBI, Pubmed and Google Scholar platforms. Any report of differential miRNA expression in a body fluid or circulating cell/particle was included, so long as the study was performed in human subjects. The studies included in the knowledge base extend as far back as 2011. The resulting knowledge base encompassed 599 reports of differential miRNA expression from 72 different studies. There were a total of 347 unique miRNA entries in the knowledge base and 253 miRNAs were recorded more than once. The miRNAs recorded more than once were either i) corroborating evidence for the miRNA expression in a single ND, ii) conflicting evidence for the miRNA expression in a single ND or iii) evidence of miRNA dysregulation across multiple NDs. All 347 miRNAs that were included in the knowledge database were deemed statistically significant by different studies in terms of *p* and False discovery rate (FDR) value (adjusted *P*-value<0.05, FDR<0.05), and the fold-change (>2 or <-2). All studies included in the construction of the knowledge database were chosen based on these criteria including that they were performed on humans with appropriately diagnosed diseased groups, and with the inclusion of appropriate control groups from healthy populations as comparators.

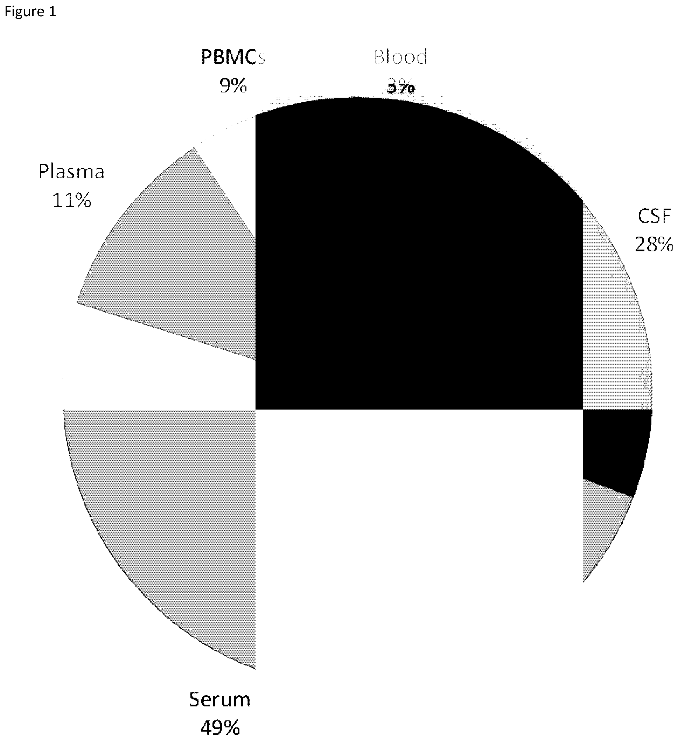

It is evident from the studies that the miRNAs were derived from diverse compartments such as blood, leukocytes, whole blood, PBMCs, plasma, serum, exosomes and CSF (Figure 1). Across all studies included, a total of 346 unique miRNAs were identified as differentially expressed (DE) in ALS, AD, PD and MS. Of the 598 reports of differential expression, 347 reported down-regulation whereas 251 reported up-regulation of miRNAs across different compartments shown in Figure 1. As evident from Figure 1, 49% of these miRNAs were analyzed from the serum compartment, 28% from the CSF, 11% from plasma, 9% from PBMCs, with the remainder coming from whole blood. Interestingly, about 17% of the miRNAs were found within membranous structures, either in exosomes or other circulating cell types. The functional similarities and differences between cell versus biofluid-derived miRNAs has not previously been analysed in this detail, especially in the context of NDs.

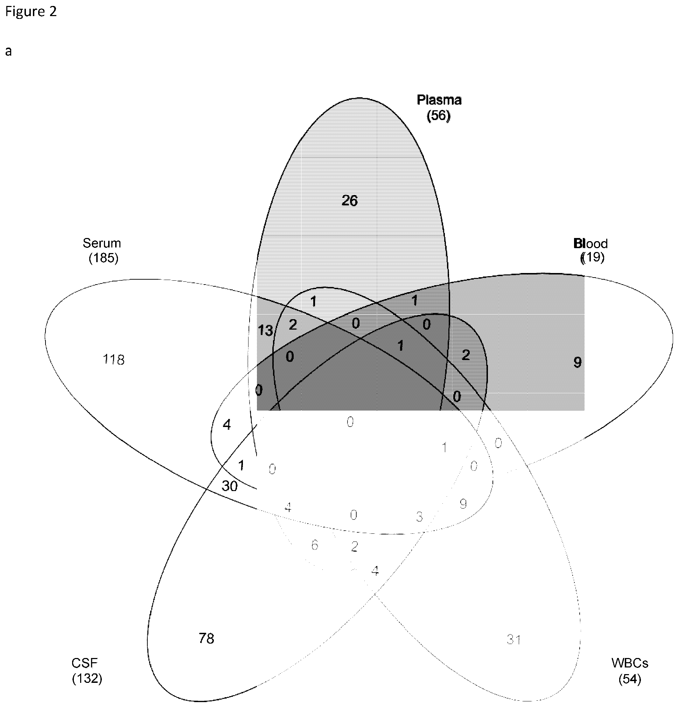

The absence of generalized overlaps between different body fluids and cellular miRNAs as shown in Figure 2a was notable from the cumulative analysis of miRNAs from all the compartments. However, inter-compartmental overlaps were observed. Moreover, miRNA specificity for each compartment was also apparent with 46% miRNA specific to plasma (26/56); 63% to Serum (118/185); 47% to Blood (9/19); 59% to the CSF (78/132) and 57% to White blood cells (WBC, 31/54) (Figure 2a). Also, when inter-compartmental relationships were visualized, the largest number of miRNAs were shared between plasma, serum and CSF, and to a lesser degree between plasma, blood and WBCs, with surprisingly no overlaps between blood and WBCs. This could be attributed to various reasons such as: i) only three studies have been done on blood as opposed to several on WBCs; ii) new miRNAs that do not have functional annotations assigned yet are not included in this analysis. Nonetheless, the difference between blood cells and WBCs is interesting, and if there are distinct miRNAs specific to WBCs, they may have important roles in immune defence and fighting off infections.

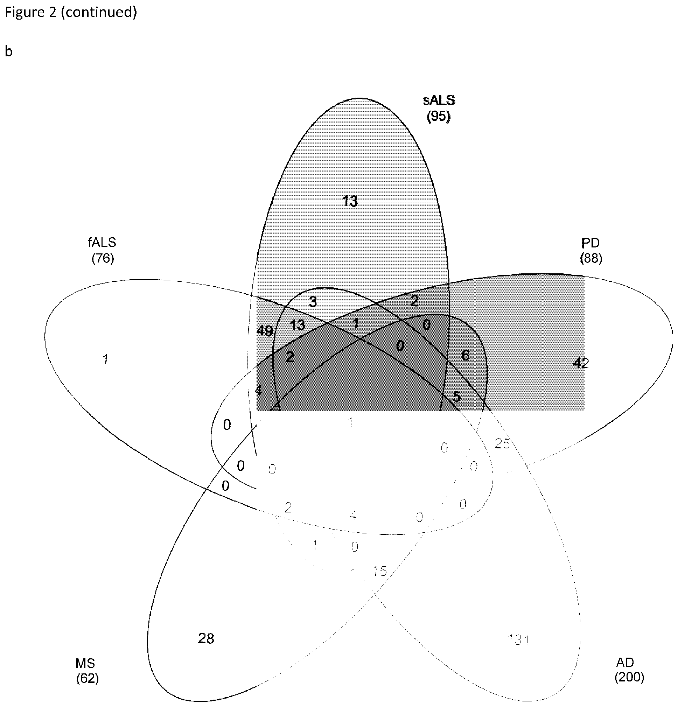

The logical relationships between miRNAs in different NDs were examined and cumulatively visualized in a Venn diagram (Figure 2b). Most of the DE miRNAs showed four different expression patterns: i) disease-specific expression miRNAs, ii) miRNA overlapping between two NDs, iii) miRNAs overlapping between three different NDs, and iv) expression intersections across all four NDs as shown in the Venn diagram (Figure 2b). These expression patterns showed both up-and-down-regulation across NDs, with the preponderance of down-regulation being the main feature of miRNA expression across NDs (58% down-regulated). Although it is highly complex to delineate the functional biological relevance of miRNAs in body fluids as opposed to cellular miRNA in the context of NDs, the data shown in Figure 2b implies that despite some overlaps between different NDs, there were miRNA unique to each ND (1 to fALS, 13 to sALS, 42 to PD, 131 to AD and 28 to MS, respectively). Thus, the disease-specific entities may also have considerable functional relevance. There was only one miRNA species identified that was differentially expressed in all four NDs. hsa-miR-30b-5p was differentially expressed in all four NDs. This is the first evidence of a miRNA species that unifies all four NDs (Figure 2b) and is a biomarker, as it encompasses some of the most relevant pathways that underlie neurodegeneration. This miRNA has also been shown to be highly abundant in the human neocortex alongside other members of the miR-30 family. Moreover, hsa-miR-30b-5p has also been shown to correlate with neurofibrillary tangles in the CSF of patients with AD and PD, along with a significant fit across increasing plaque density stages (Burgos *et al.,* 2014).

### Example 2. Generalized overlap of hsa-miR-30b-5p in neurodegenerative diseases

Although hsa-miR-30b-5p was differentially expressed in the four NDs profiled in this study, it was not differentially expressed in the same direction across all of the four NDs (Table 1). This miRNA was most frequently down-regulated in either serum or plasma across ALS, AD and PD. Interestingly, hsa-miR-30b-5p was up-regulated in MS patients in both plasma and PBMCs. The functional relevance of hsa-miR-30b-5p is significant as it is differentially expressed in all four NDs, and so is likely to be functionally relevant. Thus, the inventors looked for its cognate gene targets using DianaTools and identified KEGG pathways where these gene targets are enriched. Out of the 105 genes which were predicted to be targeted by at least two of the 346 miRNAs identified to be differentially expressed, 13 were identified to be targeted by hsa-miR-30b-5p (Figure 3) The KEGG pathways were then analyzed for relevance to neurodegenerative diseases and processes in order to derive their functional relevance in the context of the four NDs.

Examination of the KEGG pathway annotations for hsa-miR-30b-5p showed that it not only overlapped between all four NDs, but was able to target genes in multiple pathways relevant to neurodegeneration across NDs (Figure 4).

**Table 1. Expression of miR-30 family members**

| **miRNA** | **Condition** | **Expression** | **Analysis Method** | **Source** | **Chromosomal location** |
|---|---|---|---|---|---|
| **miR-30a-3p** | AD | Down | TLDA | CSF | NC_000006.12 (71403551..71403621, complement) |
| **miR-30a-3p** | PD | Down | NGS | Serum | NC_000006.12 (71403551..71403621, complement) |
| **miR-30a-5p** | PD | Up | Microarr ay & qPCR | Blood | NC_000006.12 (71403551..71403621, complement) |
| **miR-30a-5p** | PD | Up | qPCR | Plasma | NC_000006.12 (71403551..71403621, complement) |
| **hsa-miR-30b-5p** | AD | Down | Exiqon qPCR panel & qPCR | Plasma | NC_000008.11 (134800520.. 134800607, complement) |
| **hsa-miR-30b-5p** | fALS | Down | Exiqon qPCR panel & qPCR | Serum | NC_000008.11 (134800520.. 134800607, complement) |
| **hsa-miR-30b-5p** | MS | Down | NGS | Serum exosome s | NC_000008.11 (134800520.. 134800607, complement) |
| **hsa-miR-30b-5p** | PD | Up | qPCR | PBMCs | NC_000008.11 (134800520.. 134800607, complement) |
| **hsa-miR-30b-5p** | PD | Up | qPCR | Plasma | NC_000008.11 (134800520.. 134800607, complement) |
| **hsa-miR-30b-5p** | sALS | Down | Exiqon qPCR panel & qPCR | Serum | NC_000008.11 (134800520.. 134800607, complement) |
| **miR-30c** | MS | Up | Microarr ay | PBMCs | NC_000001.11 (40757284..40757372) |
| **miR-30c-2-3p** | AD | Up | NGS | Serum | NC_000006.12 (71376960..71377031, complement) |
| **miR-30c-5p** | PD | Down | Microarr ay | Serum | NC_000001.11 (40757284..40757372) |
| **miR-30d-5p** | AD | Down | TLDA | CSF | NC_000008.11 (134804876.. 134804945, complement) |
| **miR-30e-5p** | AD | Down | NGS & qPCR | Serum | NC_000001.11 (40754355..40754446) |
| **miR-30e-5p** | AD | Up | NGS & qPCR | Serum exosome s | NC_000001.11 (40754355..40754446) |
| **miR-30e-5p** | PD | Up | NGS | PBMCs | NC_000001.11 (40754355..40754446) |
| **miR-30e-5p** | PD | Down | NGS | Serum | NC_000001.11 (40754355..40754446) |

Although only hsa-miR-30b-5p overlapped between NDs, the possible functional significance and involvement of other members of the miR-30 family (hsa-miR-30b-5p, 30d-5p, 30c-p, 30e-5p, 30a-5p, 30c-2-3p, and 30a-3p) in neurodegenerative diseases was further investigated. Heatmap analysis was used to identify genes that are regulated by the members of the miR-30 family.

The heatmap analysis was performed using DIANA tools MirPath V3 (Vlachos, *et al.,* 2015). Comparative and cumulative visualization of the miR-30 family members showed critical differences between them with hsa-miR-30b-5p, 30d-5p, 30c-p, 30e-5p, and 30a-5p collectively targeting fatty acid biosynthesis pathway, the miR-30c-2-3p targeting genes in prion disease pathway, and miR30a-3p targeting both the prion and the ECM receptor interaction, implying a division of labour between -5p and -3p miRNA species at the level of their cognate targets. It appears that the miR-30 family as a whole is functionally versatile, and may therefore play a critical role in neurodegeneration.

### Example 3. Expression of hsa-miR-30b-5p in ALS patients

### miRNA Profiling

miRNA biomarker profiling was performed on total plasma samples and cerebrospinal fluid (CSF) samples obtained from seven ALS patients and two healthy control patients using the mSMRT-qPCR miRNA assay (MIRXES) in a highly controlled workflow. The miRNAs profiling process was performed in the following phases: (i) RNA was extracted from total plasma samples or CSF samples (up to 200 µl) using the Maxwell^{®} RSC miRNA Plasma and Serum (Promega, Wisconsin, United States) on a Maxwell^{®} RSC 48 instrument; (ii) a set of three proprietary spike-in control RNAs (~20 nt, MIRXES) with sequences distinct from annotated mature human miRNAs (miRbase version21) was added into the sample lysis buffer prior to RNA isolation; (iii) the quantified levels of the spike-in control RNAs were used to normalize workflow efficiency (which included RNA isolation and subsequent RT-qPCR process); (iv) the isolated miRNAs were then reverse transcribed performed on QuantStudio 5 384-well qPCR system using miRNA-specific RT primers per manufacturer's instruction (MiRXES); (v) a 6-log serial dilution of synthetic templates for each miRNA and a non-template control were concurrently reverse transcribed; (vi) sample and template cDNAs were then pre-amplified through a 15-cycle PCR reaction performed on QuantStudio 5 384-well qPCR system using Augmentation Primer Pools (MiRXES). In each amplified cDNA sample, a total of 362 candidate miRNAs were measured by qPCR using miRNA-specific qPCR assays (MIRXES), with technical replicates on ViiA7 384-well qPCR system (Applied Biosystems) and QuantStudio 5 384-well qPCR system (Applied Biosystems). Upon the completion of profiling, raw threshold cycle (Ct) values were determined using the ViiA^{™} 7 (ViiA7 384-well qPCR system) and QuantStudio^{™} Design and Analysis Software (QuantStudio 5 384-well qPCR system) RUO software with automatic baseline setting and a threshold of 0.4 and absolute copy numbers of each miRNA were determined through interpolation of the Ct values to that of the synthetic miRNA standard curves and adjusted for RT-qPCR efficiency. Technical variations introduced during RNA isolation and the process of RT-qPCR were normalized using the spike-in control RNAs.

### miRNA level Quantification

Using the standard curves derived from synthetic miRNA, the raw Cycles to Threshold (Ct) values were processed and the absolute copy numbers of the target miRNAs in each sample were determined by interpolation. Raw qPCR data was normalized in two steps. First, the technical normalization was performed to correct for the technical variations introduced during RNA isolation and RT-qPCR. This was done using the spike-in control. Next, global normalization was performed to equalize the distribution of miRNA expression across samples.

### Statistical analysis

Inter-group comparisons of miRNA levels were made using the one-tailed Wilcoxon rank sum non-parametric test. A *p*-value cut-off of 0.05 was used.

### Results

hsa-miR-30b-5p showed statistically significant higher expression levels in the CSF of the seven ALS patients compared to the two healthy control patients (Figure 5). This difference in expression in the CSF of hsa-miR-30b-5p was also evident in principal component analysis (PCA) (Figure 6), which showed a good segregation between ALS and healthy patients. These results contrast with previously reported decreases in hsa-miR-30b-5p detected in serum samples in ALS patients (Table 1 above). hsa-miR-30b-5p showed statistically significant lower expression levels in the plasma of the seven ALS patients compared to the two healthy control patients (Figures 7 and 8). Thus, the present disclosure provides a novel means of identifying ALS patients by determining hsa-miR-30b-5p expression levels in CSF, rather than serum samples.

### Example 4. hsa-miR-30b-5p in the spinal cord of ALS patients

Spinal cord samples with high RNA integrity number (RIN>7) were selected from the New York Genome database. This RNA quality control step was performed as many of the samples were taken several hours or days after the tissue donor had died, and this can result in altered gene expression and RNA degradation. Samples with high RIN at least would not suffer from significant sample degradation. Table 2 shows the breakdown of patient samples used to perform this experiment.

**Table 2. Samples and groups in analysis**

| **Tissue** | **ALS** | **Control** |
|---|---|---|
| Cervical spinal cord | 18 | 7 |
| Thoracic spinal cord | 16 | 6 |
| Lumbar spinal cord | 16 | 6 |

Genes which were differentially expressed (DEGs) in the spinal cord of ALS patients were identified. This produced a list of 2192 genes that were up or down-regulated in the spinal cords of ALS patients relative to the spinal cords of healthy controls. The genes which were separated into two lists (up-regulated genes and down-regulated genes) and submitted to the Molecular Signatures Database for further analysis (Liberzon *et al.,* 2015 and Liberzon *et al.,* 2011). Using this tool, the top 100 statistically significant predicted interactions between the input DEG lists and known, human, mature miRNAs were identified (Tables 3 and 4).

**Table 3. Cognate miRNAs (Down DEGs)**

| Gene Set Name | k/K | p-value | FDR q-value |
|---|---|---|---|
| MIR607 | 0.0571 | 9.08E-13 | 2.36E-09 |
| MIR124_3P | 0.059 | 9.58E-11 | 8.63E-08 |
| MIR506_3P | 0.0588 | 1.04E-10 | 8.63E-08 |
| MIR3671 | 0.0624 | 1.33E-10 | 8.63E-08 |
| MIR9_5P | 0.0642 | 8.41E-09 | 4.37E-06 |
| MIR4495 | 0.06 | 1.06E-07 | 4.59E-05 |
| MIR4652_3P | 0.0693 | 1.82E-07 | 6.77E-05 |
| MIR587 | 0.0632 | 9.11E-07 | 2.96E-04 |
| MIR203A_3P | 0.0618 | 1.32E-06 | 3.40E-04 |
| MIR12136 | 0.0429 | 1.39E-06 | 3.40E-04 |
| MIR95_5P | 0.049 | 1.44E-06 | 3.40E-04 |
| MIR653_5P | 0.1 | 1.60E-06 | 3.46E-04 |
| MIR340_5P | 0.0455 | 2.68E-06 | 4.75E-04 |
| MIR3688 3P | 0.0576 | 2.73E-06 | 4.75E-04 |
| MIR616_5P | 0.0427 | 2.74E-06 | 4.75E-04 |
| MIR3662 | 0.0425 | 3.03E-06 | 4.93E-04 |
| MIR548L | 0.0599 | 3.68E-06 | 5.62E-04 |
| MIR3119 | 0.059 | 4.76E-06 | 6.88E-04 |
| MIR510_3P | 0.0585 | 5.41E-06 | 7.40E-04 |
| MIR373_5P | 0.0418 | 5.74E-06 | 7.46E-04 |
| MIR371B 5P | 0.0417 | 6.18E-06 | 7.65E-04 |
| MIR627_3P | 0.0531 | 7.01E-06 | 8.28E-04 |
| MIR4795_3P | 0.0453 | 7.74E-06 | 8.75E-04 |
| MIR376C_3P | 0.0861 | 8.45E-06 | 9.15E-04 |
| MIR410_3P | 0.0601 | 9.77E-06 | 1.02E-03 |
| MIR4503 | 0.0641 | 1.13E-05 | 1.13E-03 |
| MIR302C_5P | 0.0482 | 1.22E-05 | 1.17E-03 |
| MIR875_3P | 0.0611 | 1.28E-05 | 1.18E-03 |
| MIR548N | 0.0469 | 1.41E-05 | 1.26E-03 |
| MIR3182 | 0.0654 | 1.51E-05 | 1.31E-03 |
| MIR10527_5P | 0.0471 | 1.82E-05 | 1.49E-03 |
| GTGCCTT MIR506 | 0.044 | 1.84E-05 | 1.49E-03 |
| MIR12122 | 0.0698 | 2.23E-05 | 1.76E-03 |
| MIR369_3P | 0.0598 | 2.84E-05 | 2.15E-03 |
| LET_7C_3P | 0.0468 | 2.89E-05 | 2.15E-03 |
| MIR8060 | 0.098 | 3.01E-05 | 2.17E-03 |
| MIR3658 | 0.0415 | 3.33E-05 | 2.34E-03 |
| MIR6792_3P | 0.0873 | 3.62E-05 | 2.48E-03 |
| MIR4691_5P | 0.0859 | 4.19E-05 | 2.79E-03 |
| MIR3924 | 0.0559 | 4.33E-05 | 2.82E-03 |
| MIR3923 | 0.14 | 4.82E-05 | 3.01E-03 |
| MIR380_3P | 0.0652 | 4.87E-05 | 3.01E-03 |
| MIR6844 | 0.0549 | 5.48E-05 | 3.31E-03 |
| MIR4263 | 0.0611 | 6.07E-05 | 3.55E-03 |
| MIR3147 | 0.1667 | 6.15E-05 | 3.55E-03 |
| MIR5696 | 0.0425 | 6.30E-05 | 3.56E-03 |
| MIR3675_3P | 0.0989 | 6.98E-05 | 3.86E-03 |
| MIR548AT_5P | 0.0535 | 7.70E-05 | 4.05E-03 |
| TGTTTAC_MIR30A5P_MIR30C_ MIR30D_MIR30B_MIR30E5P | 0.045 | 7.71E-05 | 4.05E-03 |
| MIR556_3P | 0.0803 | 7.80E-05 | 4.05E-03 |
| MIR93_5P | 0.0439 | 8.47E-05 | 4.31E-03 |
| MIR5688 | 0.0406 | 8.67E-05 | 4.33E-03 |
| MIR6886_3P | 0.0957 | 8.99E-05 | 4.41E-03 |
| CTCAGGG_MIR125B_MIR125A | 0.0545 | 9.28E-05 | 4.46E-03 |
| MIR1277_5P | 0.0388 | 9.82E-05 | 4.64E-03 |
| MIR582_5P | 0.0521 | 1.11E-04 | 5.10E-03 |
| MIR1297 | 0.0431 | 1.13E-04 | 5.10E-03 |
| AACTGAC_MIR223 | 0.0928 | 1.15E-04 | 5.10E-03 |
| MIR4719 | 0.0456 | 1.16E-04 | 5.10E-03 |
| MIR561_3P | 0.0463 | 1.30E-04 | 5.62E-03 |
| MIR181D_5P | 0.0433 | 1.44E-04 | 5.98E-03 |
| MIR4255 | 0.0698 | 1.44E-04 | 5.98E-03 |
| MIR181A_5P_MIR181B_5P | 0.0432 | 1.47E-04 | 5.98E-03 |
| MIR20A_5P | 0.0417 | 1.48E-04 | 5.98E-03 |
| MIR181C_5P | 0.0431 | 1.51E-04 | 5.98E-03 |
| MIR8485 | 0.0368 | 1.52E-04 | 5.98E-03 |
| MIR30A_5P | 0.0397 | 1.59E-04 | 6.13E-03 |
| MIR30D_5P | 0.0397 | 1.63E-04 | 6.13E-03 |
| MIR30E_5P | 0.0397 | 1.63E-04 | 6.13E-03 |
| MIR30B_5P_MIR30C_5P | 0.0395 | 1.78E-04 | 6.60E-03 |
| MIR106B_5P | 0.0412 | 1.84E-04 | 6.69E-03 |
| GGCAGCT_MIR22 | 0.0606 | 1.86E-04 | 6.69E-03 |
| MIR551B_5P | 0.0451 | 1.89E-04 | 6.69E-03 |
| MIR519D_3P | 0.0425 | 1.91E-04 | 6.69E-03 |
| MIR3074_5P | 0.0724 | 1.97E-04 | 6.78E-03 |
| MIR3123 | 0.0575 | 1.98E-04 | 6.78E-03 |
| MIR4328 | 0.0431 | 2.02E-04 | 6.82E-03 |
| MIR3973 | 0.0573 | 2.07E-04 | 6.89E-03 |
| MIR20B_5P | 0.0422 | 2.11E-04 | 6.92E-03 |
| MIR4666A_3P | 0.0438 | 2.13E-04 | 6.92E-03 |
| MIR7_2_3P | 0.0413 | 2.23E-04 | 7.15E-03 |
| MIR7_1_3P | 0.0412 | 2.34E-04 | 7.41E-03 |
| MIR12133 | 0.049 | 2.41E-04 | 7.48E-03 |
| MIR4324 | 0.0564 | 2.43E-04 | 7.48E-03 |
| MIR548AN | 0.0453 | 2.45E-04 | 7.48E-03 |
| MIR30A_3P | 0.0488 | 2.49E-04 | 7.50E-03 |
| MIR548T_5P | 0.0474 | 2.56E-04 | 7.50E-03 |
| MIR30E_3P | 0.0487 | 2.57E-04 | 7.50E-03 |
| MIR5582_5P | 0.0487 | 2.57E-04 | 7.50E-03 |
| MIR3668 | 0.0833 | 2.60E-04 | 7.50E-03 |
| MIR17_5P | 0.0416 | 2.63E-04 | 7.50E-03 |
| MIR1271_5P | 0.0486 | 2.66E-04 | 7.50E-03 |
| MIR30D_3P | 0.0485 | 2.74E-04 | 7.63E-03 |
| MIR122B_5P | 0.0613 | 2.78E-04 | 7.63E-03 |
| MIR3686 | 0.0515 | 2.81E-04 | 7.63E-03 |
| MIR4262 | 0.039 | 2.82E-04 | 7.63E-03 |
| MIR548AZ_5P | 0.0469 | 2.90E-04 | 7.72E-03 |
| MIR335_3P | 0.0374 | 2.96E-04 | 7.72E-03 |
| MIR4635 | 0.0746 | 2.96E-04 | 7.72E-03 |
| MIR153_5P | 0.0355 | 2.97E-04 | 7.72E-03 |

**Table 4. Cognate miRNAs (Up DEGs)**

| Gene Set Name | k/K | p-value | FDR q-value |
|---|---|---|---|
| MIR1207_5P | 0.0876 | 4.33E-09 | 1.13E-05 |
| MIR4492 | 0.0769 | 1.64E-08 | 2.12E-05 |
| MIR4498 | 0.088 | 2.89E-07 | 2.51E-04 |
| MIR4763_3P | 0.085 | 9.40E-07 | 6.11E-04 |
| MIR762 | 0.0914 | 7.22E-06 | 3.16E-03 |
| MIR4700_5P | 0.0833 | 7.29E-06 | 3.16E-03 |
| MIR765 | 0.0724 | 1.17E-05 | 3.95E-03 |
| MIR4651 | 0.0837 | 1.22E-05 | 3.95E-03 |
| MIR4667_5P | 0.0888 | 1.97E-05 | 5.68E-03 |
| CAGGGTC_MIR504 | 0.1205 | 3.60E-05 | 8.88E-03 |
| MIR3177_5P | 0.0886 | 3.76E-05 | 8.88E-03 |
| MIR5008_5P | 0.1176 | 4.43E-05 | 8.95E-03 |
| MIR6825_5P | 0.06 | 4.48E-05 | 8.95E-03 |
| MIR8089 | 0.0843 | 6.46E-05 | 1.11E-02 |
| MIR6756_5P | 0.0706 | 6.68E-05 | 1.11E-02 |
| MIR608 | 0.0762 | 6.82E-05 | 1.11E-02 |
| MIR5001_5P | 0.0828 | 7.84E-05 | 1.20E-02 |
| MIR6873_3P | 0.0602 | 8.34E-05 | 1.20E-02 |
| MIR3175 | 0.08 | 1.14E-04 | 1.56E-02 |

A Venn diagram analysis was performed on the miRNAs in the lists to ensure that the targets of these miRNAs changed in a consistent direction throughout the dataset. For example, any miRNA with numerous targets in both the up and down-regulated DEG lists is not functioning in a consistent manner and is therefore likely to be a false positive. None of the 100 miRNAs predicted to be targeting down-regulated DEGs overlapped with the 19 miRNAs predicted to target the up-regulated miRNAs (Figure 9).

Many of the miRNAs predicted to target down-regulated DEGs belong to the miR-30 family, and hsa-miR-30b-5p specifically is predicted to be targeting 31 down-regulated DEGs. Given the inventors' understanding of the importance of hsa-miR-30b-5p in ALS and neurodegenerative diseases in general (Brennan *et al.,* 2019), it is likely that this miRNA affects the disease-specific gene expression observed. Taken together, the data confirming that hsa-miR-30b-5p is up-regulated in CSF, a biofluid compartment in constant contact with the spinal cord and that hsa-miR-30b-5p and a number of related miRNAs target many of the down-regulated target genes identified in spinal tissue from ALS patients suggests this miRNA is up-regulated in a disease and tissue specific manner. The finding that many of its target genes are down-regulated in a disease and tissue specific manner is also highly congruous with the inhibitory relationship between miRNAs and their target genes. Thus, hsa-miR-30b-5p is a valuable therapeutic target for ALS patients.

### References

Brennan et al., Molecular neurobiology 56(11):7380-7407 (2019).
Burgos et al., PLoS One. 9(5):e94839 (2014).
Liberzon et al., Cell Syst 1(6):417-425 (2015).
Liberzon et al., Bioinformatics 27(12):1739-1740 (2011).
Lukiw et al., Front Genet. 3:327-327 (2013).
Vlachos et al., Nucleic Acids Research. 43(Web Server issue):W460-W466 (2015).
Zhang, L., Jia, X., Cell Mol Biol Lett 24, 61 (2019).

## Claims

1. A method of diagnosing Amyotrophic Lateral Sclerosis in a subject, the method comprising measuring the level of expression of hsa-miR-30b-5p in the cerebrospinal fluid (CSF) of the subject,
wherein an increased level of expression of hsa-miR-30b-5p relative to the level of expression of hsa-miR-30b-5p in the CSF of a healthy subject is indicative of the subject having Amyotrophic Lateral Sclerosis, and
wherein the measuring of the level of expression of hsa-miR-30b-5p in the cerebrospinal fluid (CSF) is performed on a sample comprising CSF taken from the subject.

2. The method of claim 1, wherein the level of expression of miR-30b-5p is measured by qPCR or RNA sequencing.

## Patentansprüche

1. Verfahren zum Diagnostizieren von amyotropher Lateralsklerose in einem Subjekt, wobei das Verfahren das Messen des Expressionsgrades von hsa-miR-30b-5p in der Zerebrospinalflüssigkeit (Cerebrospinal Fluid, CSF) des Subjekts umfasst,
wobei ein erhöhter Expressionsgrad von hsa-miR-30b-5p im Vergleich zum Expressionsgrad von hsa-miR-30b-5p in der CSF eines gesunden Subjekts indikativ dafür ist, dass das Subjekt an amyotropher Lateralsklerose leidet, und
wobei das Messen des Expressionsgrades von hsa-miR-30b-5p in der Zerebrospinalflüssigkeit (Cerebrospinal Fluid, CSF) an einer von dem Subjekt entnommenen CSF-haltigen Probe erfolgt.

2. Verfahren nach Anspruch 1, wobei der Expressionsgrad von miR-30b-5p durch qPCR oder RNA-Sequenzierung gemessen wird.

## Revendications

1. Procédé de diagnostic d'une sclérose latérale amyotrophique chez un sujet, le procédé comprenant une mesure du niveau d'expression de hsa-miR-30b-5p dans le fluide cérébrospinal (CSF) du sujet,
un niveau d'expression augmenté de hsa-miR-30b-5p par rapport au niveau d'expression de hsa-miR-30b-5p dans le CSF d'un sujet sain étant indicatif du fait que le sujet est atteint de sclérose latérale amyotrophique, et
la mesure du niveau d'expression de hsa-miR-30b-5p dans le fluide cérébrospinal (CSF) étant réalisée sur un échantillon comprenant du CSF prélevé du sujet.

2. Procédé selon la revendication 1, le niveau d'expression de miR-30b-5p étant mesuré par qPCR ou séquençage d'ARN.
